# EUROPÄISCHE PATENTSCHRIFT

(11) **EP 0 852 238 B1**
(45) Veröffentlichungstag und Bekanntmachung des Hinweises auf die Patenterteilung: **03.07.2002**
(21) Anmeldenummer: 97120937.4
(22) Anmeldetag: 28.11.1997
(51) Int. Cl.: C08F 246/00, C08F 220/04, C08F 230/02, C08F 228/00, A61K 31/795, A01N 25/10, A61L 17/00, A61L 31/00

(54) **Polymere mit bakteriophoben und gegebenenfalls zellproliferationsinhibierenden Eigenschaften**
Polymers with bacteriophobe and optionally cell proliferation inhibitant properties
Polymère avec des propriétésbactériophobes et éventuellement des propriétés inhibant la prolifération cellulaire

(30) Priorität: 03.01.1997 DE 19700076; 03.01.1997 DE 19700077; 03.06.1997 DE 19723132; 03.06.1997 DE 19723131
(43) Veröffentlichungstag der Anmeldung: 08.07.1998
(73) Patentinhaber: Degussa AG, 40474 Düsseldorf (DE)
(72) Erfinder: Ottersbach, Peter, Dr., 51570 Windeck (DE); Graciella, Pavon-Djavid, 92800 Puteaux (FR); Jozefowicz, Marcel, Prof, 60260 Lamorlaye (FR); Migonney, Veronique, Dr., 95600 Eaubonne (FR); Vairon, Jean-Pierre, Prof., 92340 Bourg la Reine (FR)

(56) Entgegenhaltungen:
- EP-A- 0 093 489
- EP-A- 0 604 369
- WO-A-96/39821
- US-A- 5 278 200

## Beschreibung

Die Erfindung betrifft ein Verfahren zur Herstellung von bakteriophoben und gegebenenfalls zellproliferationsinhibierenden Oberflächen unter Verwendung von bestimmten, z.T. neuen wasserunlöslichen Polymeren. Die Erfindung betrifft weiterhin die neuen, als bakteriophobe und gegebenenfalls zugleich zellproliferationsinhibierende Materialien verwendbaren Polymeren als solche sowie ein Verfahren zu deren Herstellung. Schließlich betrifft die Erfindung Erzeugnisse, die unter Verwendung der bakteriophoben und gegebenenfalls zellproliferationsinhibierenden neuen oder bekannten Materialien hergestellt werden sowie die Herstellverfahren.

### Stand der Technik

Bei allen medizinischen Untersuchungen. Behandlungen und Eingriffen, bei denen Gegenstande. Geräte oder Hilfsmittel in direktem Kontakt mit lebendem Gewebe und/oder Korperflussigkeiten kommen, können bakterielle Kontaminationen zu erheblichen Schwierigkeiten bis hin zur Gefährdung der Patienten führen. Dies trifft bei kurzen Kontakten ebenso zu wie bei mittelfristigen oder dauerhaften Applikationen von Implantaten, Kathetern, Prothesen und anderen medizintechnischen Anwendungen. So ist aus P.A. Goldmann, G.B. Pier, Clin. Microbiol. Rev. 6 (1993), 176 ff. bekannt, daß durch Infektionen bei operativen Eingriffen erhebliche und kostenintensive Komplikationen auftreten können. Aus gleichen Gründen sind Katheter nach Applikationszeiten von 2 bis 6 Tagen auszutauschen.

Auch im Bereich der Lebensmittel- und Getrankeindustrie sind Besiedelungen und Ausbreitungen von Bakterien auf Oberflächen von Rohrleitungen. Behältern oder Verpackungen in hohem Maße unerwunscht Es bilden sich haufig Schleimschichten, die Mikrobenpopulationen extrem ansteigen lassen, die Wasser-, Getränke- und Lebensmittelqualitäten nachhaltig beeinträchtigen und sogar zum Verderben der Ware sowie zu gesundheitlicher Schädigung der Verbraucher führen können.

Aus allen Lebensbereichen, in denen Hygiene von Bedeutung ist, sind Bakterien fernzuhalten. Davon betroffen sind Textilien für den direkten Körperkontakt, insbesondere für den Intimbereich und für die Kranken- und Altenpflege. Außerdem sind Bakterien fernzuhalten von Möbel- und Geräteoberflächen in Pflegestationen, insbesondere im Bereich der Intensivpflege und der Kleinstkinder-Pflege, in Krankenhäusern, insbesondere in Räumen für medizinische Eingriffe und in Isolierstationen für kritische Infektionsfälle, sowie in Toiletten

Gegenwärtig werden Geräte sowie Oberflächen von Möbeln und Textilien geger Bakterien im Bedarfsfall oder auch vorsorglich mit Chemikalien oder deren Lösungen sowie mit Mischungen behandelt, die als Desinfektionsmittel mehr oder weniger breit und massiv antimikrobiell wirken. Solche chemischen Mittel wirken unspezifisch sind häufig selbst toxisch oder reizend oder bilden gesundheitlich bedenkliche Abbauprodukte. Häufig zeigen sich auch Unvertraglichkeiten bei entsprechend sensibilisierten Personen.

Eine weitere Vorgehensweise gegen oberflächige Bakterienausbreitungen stellt die Einarbeitung antimikrobiell wirkender Substanzen in Oberflächenschichten dar, so z.B. von Silbersalzen gemäß WO 92/18098. Auch die Einarbeitung von quaternaren Ammoniumsalzen in lackartige Beschichtungen ist aus WO 94/13748 bekannt.

Derartige Komposite erweisen sich jedoch als wenig beständig gegenüber wäßrigen Lösungen oder Körperflussigkeiten. Es kommt zu Auslaugungen, wodurch die Konzentrationen der antimikrobiellen Wirkstoffe mit der Applikationsdauer reduziert und die Wirkung herabgesetzt wird. Darüber hinaus gelangen im Falle der medizinischen Anwendung derartiger Formulierungen Wirkstoffarteile in den physiologischen Kreislauf, was aufgrund toxikologischer Bedenklichkeiten und potentieller Nebenwirkungen in hohem Maße unerwünscht ist

Auf einem anderen technischen Gebiet sind aus US-PS 5 278 200 Polymere bekannt, die Carboxylat- und Sulfonatgruppen in einem dem natürlichen Heparin vergleichbaren Verhältnis enthalten. Diese Polymere weisen antikoagulierende Eigenschaften gegenüber Thrombozyten im Blut auf.

Darüber hinaus wird in vielfältiger Weise versucht, antimikrobiell wirkende Substanzen auf polymeren und/oder funktionalisierten Oberflächen, vorzugsweise durch kovalente Bindungen zu immobilisieren (T. Ouchi. Y. Ohya, Prog. Polym. Sci., 20 (1995), 211 ff.). Hierbei führt die chemische Bindung an ein Substrat im Vergleich zu den freien Wirkstoffen zu einer Herabsetzung der bakteriziden Wirkung. Bei medizinischen Anwendungen zeigen derartige Oberflächen oft Unverträglichkeiten zum physiologischen System.

Zusätzlich bleiben die abgetöteten Bakterien auf antimikrobiell wirkenden Oberflächen zurück und bauen organische Schichten auf, die schließlich die bakteriziden Wirkungen ganzlich maskieren. Die Bedeckung der Oberfläche mit abgetöteten Bakterien stellt fur den weiteren mikrobiellen Befall einen besonders vorteilhaften Nährboden dar (A. Kanazawa, T. Ikeda. T. Endo, J. Polym. Sci. A Poly. Chem. 31 (1993). 1467 ff.).

Bei bestimmten medizinischen Anwendungen kommt es nicht nur auf die bakteriophoben Eigenschaften der verwendeten Kunststoffe, sondern auch auf zellproliferationsinhibierende Eigenschaften an. Beispielsweise sind Zellbesiedlurgen bei mittelfristig intrakorporal applizierten Kathetern ebenso schädlich wie bei langfristig implantierten Stents oder Herzklappen. In WO 94/16648 wird ein Verfahren beschrieben, bei dem die Zellsorption und Zellausbreitung auf Augenimplantat-Lansen, die zur Trübung der Linse führen, durch nachtragliche chemische Modifikation der Implantat-Oberfläche verhindert wird. Gemäß EP 0 431 213 werden Polymere mit zellabweisenden Eigenschaften ausgestattet, indem die Oberflächen der Polymeren mit starken Mineralsäuren hydrophiliert werden Dies führt zu einer Verringerung der Zelladhasion.

Die nachträgliche chemische Modifizierung von Oberflächen polymerer Materialien ist meist nicht gleichmäßig und/oder einheltlich. Es bleiben häufig nicht behandelte Flächen zurück, die Ausgangspunkte für eine Zellbesiedlung der Oberfläche bilden können.

Der vorliegenden Erfindung liegt nun die Aufgabe zugrunde, ein Verfahren bereitzustellen, mit dem Oberflächen von Polymeren auf physiologisch verträgliche Weise weitgehend frei von Bakterien und gegebenenfalls zugleich von Besiedlung durch Zellen gehalten werden, ohne daß die abgetöteten Bakterien auf der Oberfläche zurückbleiben. Eine andere Aufgabe besteht darin, neue bakteriophobe und gegebenenfalls zugleich zellproliferationsinhibierende Polymere bereitzustellen. Gemäß einer anderen Aufgabe der Erfindung sollen Erzeugnisse bereitgestellt werden, die aus bestimmten bakteriophoben und gegebenenfalls zugleich zellproliferationsinhibierenden Materialien bestehen oder solche Materialien enthalten.

### Neue bakteriophobe und gegebenenfalls zellproliferationsinhibierende Polymere

Ein Gegenstand der Erfindung sind neue wasserunlösliche. bakteriophobe und gegebenenfalls zellproliferationsinhibierende Polymere B gemäß den Ansprüchen 1 bis 3 erhältlich durch radikalische Copolymerisation von
(c) einem oder mehreren Carboxylgruppen- und/oder Carboxylatgruppen-haltigen, aliphatisch ungesättigten Monomeren oder den entsprechend funktionalisierten Derivaten dieser Monomeren als Komponente I mit
(d) einem oder mehreren Sulfonsäuregruppen- und/oder Sulfonatgruppen-haltigen, aliphatisch ungesättigten Monomeren oder den entsprechend funktionalisierten Derivaten dieser Monomeren als Komponente II und
(b) einer Komponente III, die ein weiteres aliphatisch ungesättigtes Monomer oder mehrere weitere aliphatisch ungesättigte Monomere enthält.
wobei gegebenenfalls die entsprechend funktionalisierten Derivate nach der Copolymerisation zumindest an der Oberfläche in Carboxyloder Carboxylatgruppen bzw. Sulfonsäure- oder Sulfonatgruppen überführt werden.

Des weiteren ist Gegenstand der vorliegenden Erfindung ein Verfahren zur Herstellung der wasserunlöslichen, bakteriophoben Polymere B gemäß den Ansprüchen 4 bis 6 das dadurch gekennzeichnet ist, daß man
(c) ein oder mehrere Carboxylgruppen- und/oder Carboxylatgruppen-haltige, aliphatisch ungesättigte Monomere oder entsprechend funktionalisierten Derivate dieser Monomeren als Komponente I mit
(d) einem oder mehreren Sulfonsäuregruppen- und/oder Sulfonatgruppen-haltigen, aliphatisch ungesättigten Monomeren oder entsprechend funktionalisierten Derivaten dieser Monomeren als Komponente II und
- (b) .einer Komponente III, die ein weiteres aliphatisch ungesättigtes Monomer oder mehrere weitere aliphatisch ungesättigte Monomere enthält.
radikalisch copolymerisiert, wobei gegebenenfalls die entsprechend funktionalisierten Derivate nach der Copolymerisation zumindest an der Oberfläche in Carbonsäure- oder Carboxylat- bzw. Sulfonsäureoder Sulfonatgruppen überführt werden.

Die erfindungsgemäß zu verwendenden Polymeren B werden kaum von Bakterien befallen: die Adhäsion von Bakterien auf der Oberfläche wird somit hervorragend unterdrückt. Neben dieser bakteriophoben Wirkung sind die erfindungsgemäß zu verwendenden Polymeren auch bakteriostatisch, d.h. die Zellteilung der wenigen auf der Oberfläche der Polymeren adhärierten Bakterien wird stark vermindert. Dies bedeutet. daß die bakteriophobe Wirkung der erfindungsgemäßen Polymeren nicht durch das Wachstum von bereits adhärierten Bakterien maskiert werden kann. Die Reduzierung der Adsorption und der Vermehrung von Bakterien betrifft beispielsweise folgende Arten von grampositiven und gramnegativen Stämmen: Staphylococcus epidermidis, Streptococcus pyogenes, Staphylococcus aureus, Klebsiella pneumoniae, Pseudomonas aeruginosa, Escherichia coli und Enterobacter faecium.

Die besonderen Bedingungen, unter denen ein Polymer B bakteriophob und zugleich zellprolifertionsinhibierend ist, werden später erläutert werden.

Die Oberflächen der Polymeren B sind nicht nur bakteriophob und gegebenenfalls zellproliferationsinhibierend, sondern zudem ausgezeichnet mit Gewebe. Blut oder anderen Körperflüssigkeiten verträglich, da keine antimikrobiellen Wirkstoffe eingesetzt werden, die somit auch nicht in den Körper gelangen können.

Funktionalisierte Derivate der genannten Gruppen sind insbesondere Ester-, Amid- und Nitrilgruppen. Sie werden gegebenenfalls zumindest an der Oberfläche (und nur auf diese kommt es für die bakteriophobe und bakteriostatische sowie die zellproliferationsinhibierende Wirkung an) nachträglich in Carboxyl- oder Carboxylatgruppen bzw. Sulfonsäure- oder Sulfonatgruppen umgewandelt, beispielsweise durch saure oder basische Verseifung.

Bevorzugte Polymere B enthalten Carboxylat- und Sulfonatgruppen. Soweit diese Gruppen nicht durch Wahl entsprechender Monomerer eingebracht werden, kann man sie nachträglich zumindest an der Oberfläche durch Neutralisation von Carboxylgruppen bzw. Sulfonsäuregruppen mit der Lösung einer Base, wie Natronlauge, oder durch basische Verseifung von Estergruppen erzeugen.

Für die erfindungsgemäßen Polymeren B kann auch ein sowohl Carboxylund/oder Carboxylatgruppen als auch Sulfonsäure- und/oder Sulfonatgruppen enthaltendes aliphatisch ungesättigtes Monomer eingesetzt werden. Solche bifunktionellen Monomere fungieren gleichzeitig als Komponenten I und II. Alternativ können jeweils mehrere Carboxylund/oder Carboxylatgruppen oder Sulfonsäureund/oder Sulfonatgruppen enthaltende aliphatisch ungesättigte Monomere oder entsprechend funktionalisierte Derivate dieser Monomeren verwendet werden.

Zweckmäßig beträgt für die erfindungsgemäßen Polymeren 8 die Summe der Anteile der Komponente I und der Komponente II 0.5 bis 30 Mol-% des Polymeren.

Vorteilhaft beträgt das Verhältnis der im erfindungsgemäßen Polymer B enthaltenen Carboxyl- und/oder Carboxylatgruppen zu Sulfonsäure und/oder Sulfonatgruppen 0.5 bis 10, vorzugsweise 0.5 bis 5. Wenn das genannte Verhältnis 0,4 bis 3, vorzugsweise 0,4 bis 2 beträgt, zeigen die Polymeren B zudem ausgeprägte zellproliferationsinhibierende Eigenschaften.

### Monomere für die Polymeren B

Als Komponenten I und II für die Herstellung der erfindungsgemäßen Polymeren B eignen sich z.B. Monomere der allgemeinen Formeln I und II.

Formel I (CₙH_{2n-q-x})(COOR¹)ₓ (für Komponente I)

Formel II (CₙH_{2n-q-x})(SO₃R¹)ₓ (für Komponente II)

die bevorzugte Monomere für die Herstellung der Polymeren B sind. In den Formeln I und II tehen
- n: jeweils unabhängig für eine ganze Zahl von 2 bis einschließlich 6;
- q: jeweils unabhängig für 0 oder 2.
- x: jeweils unabhängig für 1 oder 2; und
bedeutet der Rest R¹ jeweils unabhängig -H, ein Äquivalent eines Metallions, insbesondere ein Alkalimetallion, oder einen Rest eines aliphatischen, cycloaliphatischen oder araliphatischen Alkohols, vorzugsweise eines Alkanols mit 1 bis 6, insbesondere mit 1 bis 4 Kohlenstoffatomen, eines Cycloalkanols mit 5 bis 12 Kohlenstoffatomen, eines Arylalkanols mit 7 bis 10 Kohlenstoffatomen oder eines Alkanols mit Sauerstoff- und bzw. oder Stickstoffatomen in der Kette und bis zu 12 Kohlenstoffatomen.

Soweit die Gruppen (COOR¹)ₓ und (SO₃R¹)ₓ Estergruppen sind, werden sie nach der Polymerisation durch Verseifung in Carboxyl- oder Carboxylatgruppen bzw. Sulfnosäure- oder Sulfonatgruppen umgewandelt.

Den gegebenen Definitionen entsprechend bedeutet der Rest (CₙH_{2n-q-x})- jeweils unabhängig einen geradkettigen oder verzweigten einwertigen Alkenylrest (q=0, x=1) oder Alkadienylrest (q=2, x=1) oder einen geradkettigen oder verzweigten zweiwertigen Alkenylenrest (q=0, x=2) oder Alkadienylenrest (q=2, x=2).

Anstelle von zwei Monomeren der Formeln I und II kann man wiederum auch nur ein bifunktionelles Monomer (I + II) einsetzen, das die Gruppen COOR¹ und SO₃R¹ in demselben Molekül enthält.

Als Spezialfall von Monomeren, die sich als Komponente I eignen. seien die folgenden, unter die Formel I fallenden Monomere der Formel III genannt:

Formel III: (CₙH_{2n-q-x}) (COOR²)ₓ .

wobei
- R² =: -(CH₂-CH₂-O)_{d}-H. -(CH₂-CH(CH₃)-O)_{d}-H, -(CH₂-CH₂-CH₂-O)_{d}-H oder -(CH₂)_{d}-NH₂₋ₑ(R³)ₑ bedeuten, wobei R³ = -CH₃ oder -C₂H₅ bedeutet. d = 0, 1, 2, 3 oder 4 und e = 0, 1 oder 2 bedeutet.
- n =: 2, 3, 4, 5 oder 6,
- q =: 0 oder 2,
- x =: 1 oder 2 und
bedeuten.

Die Carbonestergruppen werden nach der Polymerisation verseift und liegen dann als Carboxyl- oder Carboxylatgruppen vor. Die aliphatisch ungesättigten Monomeren können sowohl geradkettig als auch verzweigt sein.

Als Spezialfall von Monomeren, die sich als Komponente I eignen seien die folgenden, unter die Formel II fallenden Monomere der allgemeinen Formel IV genannt:

Formel IV (CₙH_{2n-q-x}) (SO₃R²)ₓ,

in der R², n, q und x die angegebenen Bedeutungen haben.

Die Sulfonestergruppen werden nach der Polymerisation verseift und liegen dann als Sulfonsäure- oder Sulfonatgruppen vor. Die aliphatisch ungesättigten Monomeren können sowohl geradkettig als auch verzweigt sein.

Auch vom Benzol abgeleitete Monomerkomponenten der allgemeinen Formel, V

Formel V : (C₆H_{6-a-b-c})AₐB_{b}(OH)_{c}

eignen sich als Komponenten I und II für das Polymer B, wobei
- A: jeweils unabhängig einen ein- oder zweiwertigen geradkettigen oder verzweigten Rest der Formeln -(CₙH_{2n-1-q-x})(COOR¹)ₓ oder -(CₙH_{2n-1-q-x})(SO₃R¹)ₓ bedeutet, in denen R¹, n, q, x und y wie zuvor definiert sind:
- B: jeweils unabhängig C₁₋₄-Alkyl, -NH₂, -COOH, -SO₃H, -OSO₃H, -OPO(OH)₂, -PO(OH)₂, -OP(OH)₂, -OPO(O⁻)OCH₂CH₂N⁺(CH₃)₃, -PO(O⁻)OCH₂CH₂N⁺(CH₃)₃, -OP(O-)OCH₂CH₂N⁺(CH₃)₃ oder gegebenenfalls ein Salz, insbesondere ein Alkalisalz, oder einen Ester der genannten Gruppen bedeutet:
- a: für 1. 2 oder 3 steht:
- b: für 0, 1. 2 oder 3 steht; und
- c: für 0, 1, 2 oder 3 steht;
mit der Maßgabe. daß a + b + c ≤ 6, vorteilhaft ≤4 ist.

Je nach der Bedeutung von A sind die Monomeren der Formel V als Komponente I oder als Komponente II geeignet.

Als Spezialfall von Monomeren, die unter die allgemeine Formel V fallen und sich als Komponente I eignen, seien die folgenden Monomeren der allgemeinen Formel VI genannt:

Formel VI : (C₆H_{6-a-b-c})CₐD_{b}(OH)_{c},

in der bedeuten

C = (CₙH_{2n-q-x-1})(COOR²)ₓ,

wobei
- R² =: -(CH₂-CH₂-O)_{d}-H, -(CH₂-CH(CH₃)-O)_{d}-H, - (CH₂-CH₂-CH₂-O)_{d}-H oder -(CH₂)_{d}-NH₂₋ₑ(R³)ₑ bedeutet, wobei R³ = -CH₃ oder -C₂H₅ bedeutet,
n = 2, 3, 4, 5 oder 6,
q = 0 oder 2,
x = 1 oder 2,
d = 0, 1, 2, 3 oder 4 und
e = 0, 1 oder 2 bedeuten;
- D =: -COOH, -SO₃H, -NH₂, -N+(CH₃)₃, -O-PO₃H -. -OSO₃H. oder -O-PO₂⁻-CH₂-CH₂-N+(CH₃)₃,
- a =: 1, 2 oder 3,
- b =: 0, 1, 2 oder 3 und
- c =: 0, 1, 2 oder 3;
mit der Maßgabe, daß a + b + c ≤ 6, vorteilhaft ≤4 ist.

Als Spezialfall von Monomeren, die unter die allgemeine Formel V fallen und sich sich als Komponente II eignen, seien die folgenden, Monomeren der allgemeinen Formel VII genannt:

Formel VII (C₆H_{6-a-b-c})EₐD_{b}(OH)_{c}.

in der bedeuten:

E = (CₙH_{2n-q-x-1})(SO₃R²)_{y},

wobei
- R² =: -(CH₂-CH₂-O)_{d}-H, -(CH₂-CH(CH₃)-O)_{d}-H, - (CH₂-CH₂-CH₂-O)_{d}-H oder -(CH₂)_{d}-NH₂₋ₑ(R³)ₑ wobei R³ = -CH₃ oder -C₂H₅ bedeutet.
n = 2, 3, 4, 5 oder 6,
q = 0 oder 2,
x = 1 oder 2,
d = 0, 1, 2, 3 oder 4 und
e = 0, 1 oder 2 bedeuten;
- D =: -COOH, -SO₃H, -NH₂, -N+(CH₃)₃, -O-PO₃H -, -OSO₃H, -O-PO₂⁻ -CH₂-CH₂-N⁺ (CH₃)₃
- a =: 1, 2 oder 3.
- b =: 0. 1, 2 oder 3 und
- c =: 0, 1, 2 oder 3;
mit der Maßgabe. daß a + b + c ≤ 6. vorteilhaft ≤4 ist.

Auch in den beiden letztgenannten Spezialfällen werden Estergruppen nach der Polymerisation verseift und liegen dann als die entsprechenden sauren oder ionischen neutralisierten Gruppen vor.

Von den für die Herstellung der Beschichtungspolymeren geeigneten Monomeren der allgemeinen Formeln I bis VII die einen oder mehrere gleiche oder verschiedene, tatsächliche oder potentielle (d.h. nach Verseifung tatsächliche) Reste A im Molekül enthalten, seien beispielsweise genannt:
Acrylsäure, Natriumacrylat, Isobutylacrylat, 2-Ethylhexylacrylat. 2-Hydroxyethylacrylat, 2-(2'-Hydroxyethoxy)ethylacrylat, 2-Hydroxy-1-methylethylacrylat, 2-N,N-Dimethylaminoethylacrylat, Methacrylsäure, Natriummethacrylat, n-Propylmethacrylat, 2-Hydroxyethylmethacrylat, 2-(2'-Hydroxyethoxy)ethylmethacrylat, 2-Hydroxy-1-methylethylmethacrylat, 2-N,N-Dimethylaminoethylmethacrylat. Maleinsäure, Diethylenglykolmethacrylat, Triethylenglykoldiacrylat, Natriumallylsulfat, Natriummethallysulfat, 2-Hydroxyethylallylsulfat. Vinylsulfonsäure, Natriumvinylsulfonat, Vinylsulfonsäure-2-hydroxyethylester, Vinylbenzolsulfonsäure, Natriumvinyltoluylsulfonat, 4-Vinylsalicylsäure. Butadien-(1,3)-diol-(1,4)-diphosphat. Sorbinsäure. Coffeinsäure, 4- und 2-Vinylphenol, 2-Allylhydrochinon, 4-Vinylresorcin, Carboxyl-styrolsulfonsäure.

### Weitere Monomere

Als weitere aliphatisch ungesättigte Monomere (Komponente III) eignen sich für die Polymeren B vorzugsweise Monomere, die keine ionischen Gruppen tragen. Die aliphatisch ungesättigten Monomeren tragen überwiegend dazu bei, daß die Polymeren 3 wasserunlöslich sind. Durch orientierende Versuche läßt sich unschwer ermitteln, welche Monomere in welcher Menge als Komponente III eingesetzt werden können, damit die Polymeren B wasserunlöslich sind. Als wasserunlöslich werden sie dann angesehen. wenn sie sich im Kontakt mit wäßrigen Medien bei bestimmungsgemäßem Gebrauch der aus ihnen hergestellten bzw. der mit ihnen beschichteten Erzeugnisse auch nach Monaten nicht sichtbar verändern. Zu den im Prinzip als aliphatisch ungesättigte Monomere geeigneten Monomeren gehören beispielsweise Vinylverbindungen, z.B. Vinylketone, wie Vinylethylketon und Vinyl-n-butylketon; Vinylester, wie vinylacetat und -propionat; Allylverbindungen: (Meth)acrylverbindungen, wie (Meth)acrylsäure ester, z.B. Methylacrylat, Methacrylmethacrylat, n-Butylacrylat und 2-Ethylhexylacrylat; ferner Acrylnitril und Acrylamid; Olefine und Diene, wie 1-Buten, 1-Hexen, 1,3-Butadien. Isopren und Chloropren; ungesättigte Halogenkohlenwasserstoffe, wie Vinylchlorid und Vinylidenchlorid; Vinylsiloxane, wie Tris(trimethylsiloxy)methacryloylsilan und Tris(trimethylsiloxy)acryloylpropylsilan; bzw. deren entsprechend funktionalisierte Derivate. Wenn und soweit Carbonester-, Nitril- und Carbonamidgruppen nicht nachträglich hydrolysiert werden, gelten die entsprechenden Monomeren als aliphatisch ungesättigte Monomere (Komponente III).

### Herstellung der bakteriophoben und gegebenenfalls zellproliferationsinhibierenden Polymeren B

Die erfindungsgemäß zu verwendenden bzw. erfindungsgemäßen Polymeren B können beispielsweise mit Hilfe der Emulsionspolymerisation nach dem Stand der Technik hergestellt werden (Hans-Georg Elias. Makromoleküle. Hüthig & Wepf Verlag, Heidelberg, 1981, S. 603 ff.).

Weiterhin können zur Herstellung der erfindungsgemäßen Polymeren B die Komponenten I, II und III auch in Lösung oder in Substanz nach bekannten Verfahren copolymerisiert werden (Hans-Georg Elias, Makromoleküle, Hüthig & Wepf Verlag, Heidelberg, 1981, S. 602 ff.).

Zur Copolymerisation der Komponenten I, II und III in Lösung können beispielsweise, je nach eingesetzten Monomeren, folgende Lösemittel eingesetzt werden: Wasser, Ketone, wie Aceton, Methylethylketon, Butanon und Cyclohexanon; Ether, wie Diethylether, Tetrahydrofuran und Dioxan; Alkohole, wie Methanol, Ethanol, n- und iso-Propanol, n-und iso-Butanol und Cyclohexanol; stark polare Lösemittel, wie Dimethylacetamid, Dimethylsulfoxid und Dimethylformamid; Kohlenwasserstoffe, wie Heptan, Cyclohexan, Benzol und Toluol; Halogenkohlenwasserstoffe, wie Dichlormethan und Trichlormethan; Ester, wie Ethylacetat, Propylacetat und Amylacetat; sowie Nitrile, wie Acetonitril.

Als Polymerisationsinitiatoren lassen sich u. a. Azonitrile, Alkyl peroxide, Acylperoxide, Hydroperoxide, Peroxoketone, Perester und Peroxocarbonate, Peroxodisulfat, Persulfat sowie alle üblichen Photoinitiatoren verwenden. Die Polymerisationsinitiierung kann thermisch oder durch elektromagnetische Strahlung, wie UV-Licht oder gamma-Strahlung, erfolgen.

Werden zur Herstellung der bakteriophoben und gegebenenfalls zellproliferationsinhibierenden Polymeren aus Monomeren der Formeln I bis VII keine Carboxyl- und/oder Carboxylatgruppen-haltige oder Sulfonsäure- und/oder Sulfonatgruppen-haltigen Monomere eingesetzt. sondern deren funktionalisierte Derivate, z.B. ein Carbonsäureester anstelle einer Carbonsäure, so müssen die funktionalisierten Derivate nach der Polymerisation in Carboxyl- bzw. Carboxylatgruppen und bzw. oder Sulfonsäure- bzw. Sulfonatgruppen überführt werden. Dies kann im Falle der Ester mittels sauer oder basisch katalysierter Verseifung erfolgen. Die Derivatisierung von polymeren Materialien kann nach allgemein bekannten Verfahren (Hans Beyer. Lehrbuch der organischen Chemie, S. Hirzel Verlag. Stuttgart, 1988, S. 260 ff.) vorgenommen werden.

### Verwendung der Polymeren

Ein weiterer Gegenstand der vorliegenden Erfindung ist die Verwendung der wasserunlöslichen Polymeren B nach den Ansprüchen 1 bis 3 zur Herstellung von Erzeugnissen mit bakteriophober und gegebenenfalls zellproliferationsinhibierender Oberfläche gemäß den Ansprüchen 9 bis 11. Dazu gehören Erzeugnisse, die ganz oder teilweise aus diesen Polymeren bestehen, und Erzeugnisse aus Kunststoff. Keramik oder Metall, die mit den Polymeren beschichtet sind. Zur Beschichtung eignen sich die bekannten Verfahren, wie Tauchen, Spritzen, Streichen, Rakeln und Spin-Coating. Die Beschichtung mit den Polymeren nach der Erfindung macht es möglich, bekannte und bewährte Materialien und Herstellverfahren weiterzuverwenden. Dies ist insbesondere dann interessant, wenn die mechanischen Eigenschaften der Werkstoffe von hoher, Bedeutung sind oder die bestehenden Fertigungsanlagen nach den bisherigen Herstellverfahren hohe Investitionen erforderten.

Weiterhin ist eine Fixierung der Polymeren durch Primerschichten oder Zwischenschichten aus bifunktionellen Verbindungen auf gegebenenfalls aktivierten Standardpolymeren möglich. Derartige Standardpolymere sind beispielsweise PVC, Polystyrol, Polyurethane, Polyacrylate. Polymethacrylate, Polyester, Polyether, Polyetherblockamide, Polyamide, Polycarbonate, Polyolefine, Silicone und Polytetrafluorethylen.

Die erfindungsgemäß hergestellten Erzeugnisse mit bakteriophober und gegebenenfalls zellproliferationsinhibierender Oberfläche sind vorzugsweise zur Verwendung auf den Gebieten der Nahrungs- und Genußmitteltechnik, Wassertechnik, Biotechnik, der Hygienevorsorge und insbesondere der Medizintechnik bestimmt. Beispielsweise lassen sich die hier beschriebenen Polymeren verwenden zur Herstellung von Erzeugnissen, wie Textilien, Möbeln und Geräten, Rohren und Schläuchen, Fußböden, Wand- und Deckenflächen. Lagerbehältern, Verpackungen, Fensterrahmen, Telefonhörern, Toilettensitzen, Türgriffen; Griffen und Haltegurten in öffentlichen Verkehrsmitteln und von medizintechnischen Artikeln, die aus den Polymeren bestehen oder eine Beschichtung mit diesen Polymeren auf Kunststoffen, Keramiken oder Metall als Substrat aufweisen. Medizintechnische Artikel sind beispielsweise Implantate oder Hilfsmittel, wie Drainagen, Führungsdrähte, Kanülen, Intraokularlinsen, Kontaktlinsen, Stents, Gefäßprothesen, Gelenkprothesen, Knochenersatzmaterialien, Bandprothesen, Zahnprothesen für die plastische Chirurgie, Blutbeutel, Dialysemembranen, Nahtmaterialien, Verbandstoffe, Vliesprodukte und Operationsbestecke. Eine bevorzugte Verwendung der Materialien nach der Erfindung ist die zur Herstellung von Kathetern.

### Erfindungsgemäße Erzeugnisse

Schließlich sind Gegenstand der vorliegenden Erfindung die Erzeugnisse gemäß den Ansprüchen 12 bis 16 die unter Verwendung der wasserunlöslichen, bakteriophoben und gegebenenfalls zellproliferationsinhibierenden Polymeren gemäß den Ansprüchen 1 bis 8 hergestellt wurden, wie sie zuvor beschrieben wurden, insbesondere die genannten medizintechnischen Erzeugnisse und vorzugsweise Katheter.

Die folgenden Beispiele sollen die Erfindung näher erläutern, nicht aber ihren Umfang begrenzen, wie er in den Patentansprüchen definiert ist.

### Beispiele

### Testverfahren zur Bakterienadhäsion

### Messung der Bakterienadhäsion über Szintillation

Die durch Copolymerisation erhaltenen Proben des jeweiligen erfindungsgemäßen Polymers werden in einem geeigneten Lösemittel gelöst. Nach Ausgießen in eine Petrischale und Verdampfung des Lösemittels werden die erhaltenen Polymerfolien über einen Zeitraum von einer Stunde in 1 ml einer Lösung eingetaucht, die aus Phosphat-gepufferter Saline (PBS), 0.4 g/l Rinderserumalbumin (BSA) und 20 µg/ml gereinigtem humanen Fibronektin besteht. Anschließend werden die so mit Fibronektin beschichteten Proben unter kräftigem Rühren 1 Stunde lang bei 37°C in die entsprechende Bakteriensuspension gegeben, die durch den Einbau von 3H-Thymidin radioaktiv markiert worden ist. Nach Ablauf der vorgegebenen Zeitspanne werden die überschüssigen Bakterien entfernt, die Membranen 3 mal mit jeweils 3 ml einer PBS-BSA-Lösung abgespült und zur Bestimmung der Anzahl adhärierter Bakterien in eine Probenkammer mit 20 ml Szintillationslösung gelegt. Der Prozentsatz an adhärierten Bakterien wird über das Verhältnis der von der Probe ausgehenden zur Anzahl der von den Bakterien ursprünglich eingebrachten Radioaktivität bestimmt. Als Referenzprobe dient eine auf die gleiche Weise vorbereitete Folie, die durch Polymerisation der Komponente III des jeweiligen erfindungsgemäßen Polymers erhalten wird. Die Hemmung der Bakterienadhäsion wird als prozentualer Quotient der Bakterienadhäsion der Referenzfolie zu der des erfindungsgemäßen Polymeren bestimmt.

### Messung der Bakterienadhäsion beschichteter Standardfolien über ATP (statisch)

Nach einer Adsorption von Mikrobenzellen an Folien aus dem jeweiligen erfindungsgemäßen Polymer werden die nichtadhärierten Bakterien mit steriler PBS-Pufferlösung weggespült. Aus den an der Folie adhärierenden Bakterien wird der Bakterieninhaltsstoff Adenosintriphosphat (ATP) extrahiert und mit einer handelsüblichen Testkombination im bioluminometrischen Test bestimmt. Die Anzahl der gemessenen Lichtimpulse ist proportional zur Zahl an adhärierenden Bakterien. Da der ATP-Gehalt der verschiedenen Bakterienstämme unterschiedlich ist, werden in jedem Fall mehrere Standardfolien eingesetzt. Als Referenzprobe dient eine auf die gleiche Weise vorbereitete Folie, die durch Polymerisation der Komponente III des jeweiligen erfindungsgemäßen Polymers erhalten wird. Die Hemmung der Bakterienadhäsion wird als prozentualer Quotient der Bakterienadhäsion der Referenzfolie zu der des erfindungsgemäßen Polymeren bestimmt.

### Messung der Bakterienadhäsion beschichteter Standardfolien über ATP (dynamisch)

Die Bakterien werden mit der zu prüfenden Folie aus dem jeweiligen erfindungsgemäßen Polymer in eine Hefeextrakt-Pepton-Glucose-Nährlösung gegeben und 24 Stunden bei 37°C geschüttelt. Im Anschluß wird das Kunststoffstück mit Leitungswasser gespült, in einen frischen Kolben mit Nährlösung übertragen und für weitere 24 Stunden bei 37°C geschüttelt. Daraufhin wird der letzte Vorgang noch einmal wiederholt, das Folienstück wird mit Leitungswasser gespült und in sterile PBS getaucht. Aus den an der Folie adhärierenden Bakterien wird der Zellinhaltsstoff Adenosintriphosphat (ATP) extrahiert und mit einer handelsüblichen Testkombination im bioluminometrischen Test bestimmt. Dieser Wert ist proportional zu den adhärierten Bakterien. Als Referenzprobe dient eine auf die gleiche Weise vorbereitete Folie, die durch Polymerisation der Komponente III des jeweiligen erfindungsgemäßen Polymers erhalten wird. Die Hemmung der Bakterienadhäsion wird als prozentualer Quotient der Bakterienadhäsion der Referenzfolie zu der des erfindungsgemäßen Polymeren bestimmt.

Die in den nachfolgenden Beispielen aufgeführten Meßergebnisse zeigen, daß die Adhäsion von Bakterien an erfindungsgemäßen Polymeren unabhängig von Bakterienstamm und Meßmethode 95 bis 99 % beträgt. also nahezu vollständig ist. Diese Wirkung wird auch nicht durch abgestorbene Mikroorganismen maskiert.

### Meßverfahren zur Bestimmung der Zellproliferation

### Herstellung der Zellsuspension

Menschliche Fibroblasten (McCoy's) von ATCC No.CRL 1696 (Rockville, Maryland. USA) werden in einem DMEM-Medium (Dulbecco's Modified Eagles Medium) unter Zusatz von Antibiotika, L-Glutamin und 10 Vol.-% eines fötalen Kälberserums bei 37°C unter einer Atmosphäre von 5 % CO₂ und 95 % Luft gezüchtet. Nach Abtrennung der Zellen vom Nährmedium wird sowohl die Anzahl lebender Zellen mit dem MTT-Färbetest (MTT = 3-(4,5-Dimethylthiazol-2-yl)-2,5-diphenyltetrazoliumbromid) als auch deren Gesamtanzahl bestimmt.

### Messung der Zellinhibierung

Proben des jeweiligen erfindungsgemäßen Polymers werden in Wells (Vertiefungen von Standardmikrotiterplatten) gegeben und mittels spezieller PTFE-Einsätze, die zuvor mit Ethanol sterilisiert werden. arretiert. Proben, Wells und PTFE-Einsätze werden zusätzlich durch 15-minütige Bestrahlung mit ultraviolettem Licht sterilisiert. Anschließend werden die Polymerproben mit der Zellsuspension bekannter Konzentration versetzt und bei 37°C in einer Atmosphäre von 95 % Luft und 5 % CO₂ gehalten. Nach Ablauf von acht Tagen werden die Zellen mittels einer Phosphatpufferlösung gewaschen, in 0.05 Gew.-% Trypsin-Lösung und 0.02 Gew.-% EDTA-Lösung bei pH 7.4 suspendiert und mit Hilfe eines Coulter-Counters ausgezählt.

Als Referenzprobe dient eine auf die gleiche Weise vorbereitete Probe, die durch Polymerisation der Komponente III des jeweiligen erfindungsgemäßen Polymeren erhalten wird. Die Inhibierung der Zellproliferation wird als prozentualer Quotient der Anzahl der Zellen auf den Referenzproben zu der auf den erfindungsgemäßen Polymeren bestimmt.

### Herstellung von Proben der erfindungsgemäßen Polymeren

### Beispiel 1:

In einer Stickstoffatmosphäre werden 218.2 g Methacrylsäuremethylester, 12.1 g Methacrylsäure und 14.8 g Natriumstyrolsulfonat in 500 ml Dimethylsulfoxid gelöst. Die Lösung wird unter Rühren auf 70 C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 16 Stunden bei 70°C geführt. Im Anschluß wird das entstandene Produkt in einem vierfachen Über-schuß Eiswasser gefällt, daraufhin 24 Stunden im Soxhlet mit Wasser extrahiert und bei 50°C im Vakuum getrocknet. Eine anschließende Analyse der Zusammensetzung über 1H-NMR ergibt:

| | |
|---|---|
| Methacrylsäure | 14 Mol-% |
| Natriumstyrolsulfonat | 11 Mol-% |
| Methacrylsäuremethylester | 75 Mol-% |

Aus diesen Werten ergibt sich ein Verhältnis der Carboxylatgruppen zu Sulfonatgruppen von 1.27.

### Beispiel 2:

In einer Stickstoffatmosphäre werden 216.3 g Methacrylsäuremethylester. 13.8 g Acrylsäure und 9.9 g Natriumstyrolsulfonat in 500 ml Dimethylsulfoxid gelöst. Die Lösung wird unter Rühren auf 70°C erhitzt. Anschließend werden 2 g Azobisisobutyronitril gelöst in 30 ml Dimethylsulfoxid innerhalb von 2 Minuten zugetropft. Die Polymerisation wird über einen Zeitraum von 16 Stunden bei 70°C geführt. Im Anschluß wird das entstandene Produkt in einem vierfachen Überschuß Eiswasser gefällt, daraufhin 24 Stunden im Soxhlet mit Wasser extrahiert und bei 50°C im Vakuum getrocknet.
Eine anschließende Analyse der Zusammensetzung über 1H-NMR ergibt:

| | |
|---|---|
| Acrylsäure | 10 Mol-% |
| Natriumstyrolsulfonat | 9 Mol-% |
| Methacrylsäuremethylester | 81 Mol-% |

Aus diesen Werten ergibt sich ein Verhältnis der Carboxylatgruppen zu Sulfonatgruppen von 1.11.

### Beispiel 3:

In einer Stickstoffatmosphäre werden 235 g Styrol, 6.1 g Methacrylsäure und 14.8 g Natriumstyrolsulfonat in 500 ml Dimethylsulfoxid gelöst. Die Lösung wird unter Rühren auf 70°C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 16 Stunden bei 70°C geführt. Im Anschluß wird das entstandene Produkt in einem vierfachen Überschuß Eiswasser gefällt, daraufhin 24 Stunden im Soxhlet mit Wasser extrahiert und bei 50°C im Vakuum getrocknet.
Eine anschließende Analyse der Zusammensetzung über 1H-NMR ergibt:

| | |
|---|---|
| Methacrylsäure | 15 Mol-% |
| Natriumstyrolsulfonat | 7 Mol-% |
| Styrol | 78 Mol-% |

Aus diesen Werten ergibt sich ein Verhältnis der Carboxylatgruppen zu Sulfonatgruppen von 2.14.

### Beispiel 4:

In einer Stickstoffatmosphäre werden 227.5 g Styrol, 8.1 g Acrylsäure und 24.7 g Natriumstyrolsulfonat in 500 ml Dimethylsulfoxid gelöst. Die Lösung wird unter Rühren auf 70°C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 20 Stunden bei 70°C geführt. Im Anschluß wird das entstandene Produkt in einem vierfachen Überschuß Eiswasser gefällt, daraufhin 24 Stunden im Soxhlet mit Wasser extrahiert und bei 50°C im Vakuum getrocknet.
Eine anschließende Analyse der Zusammensetzung über 1H-NMR ergibt:

| | |
|---|---|
| Acrylsäure | 11 Mol-% |
| Natriumstyrolsulfonat | 12 Mol-% |
| Styrol | 77 Mol-% |

Aus diesen Werten ergibt sich ein Verhältnis von Carboxylatgruppen zu Sulfonatgruppen von 0.9.

### Beispiel 5:

In einer Stickstoffatmosphäre werden 310.0 g n-Butylmethacrylat, 12.1 g Methacrylsäure und 14.8 g Natriumstyrolsulfonat in 500 ml Dimethylsulfoxid gelöst. Die Lösung wird unter Rühren auf 70°C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 20 Stunden bei 70°C geführt.
Im Anschluß wird das entstandene Produkt in einem vierfachen Überschuß Eiswasser gefällt, daraufhin 24 Stunden im Soxhlet mit Wasser extrahiert und bei 50°C im Vakuum getrocknet.
Eine anschließende Analyse der Zusammensetzung über 1H-NMR ergibt:

| | |
|---|---|
| Methacrylsäure | 14 Mol-% |
| Natriumstyrolsulfonat | 9 Mol-% |
| n-Butylmethacrylat | 77 Mol-% |

Aus diesen Werten ergibt sich ein Verhältnis der Carboxylatgruppen zu Sulfonatgruppen von 1.6.

### Beispiel 6

In einer Stickstoffatmosphäre werden 306.7 g n-Butylmethacrylat, 13.8 g Acrylsäure und 9.9 g Natriumstyrolsulfonat in 500 ml Dimethylsulfoxid gelöst. Die Lösung wird unter Rühren auf 70°C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 16 Stunden bei 70°C geführt. Im Anschluß wird das entstandene Produkt in einem vierfachen Überschuß Eiswasser gefällt, daraufhin 24 Stunden im Soxhlet mit Wasser extrahiert und bei 50°C im Vakuum getrocknet.
Eine anschließende Analyse der Zusammensetzung über 1H-NMR ergibt:

| | |
|---|---|
| Acrylsäure | 10 Mol-% |
| Natriumstyrolsulfonat | 8 Mol-% |
| n-Butylmethacrylat | 82 Mol-% |

Aus diesen Werten ergibt sich ein Verhältnis von Carboxylatgruppen zu Sulfonatgruppen von 1.3.

### Beispiel 7:

In einer Stickstoffatmosphäre werden 220 g Styrol, 15.6 g Acrylsäure und 14.8 g Natriumstyrolsulfonat in 500 ml Dimethylsulfoxid gelöst. Die Lösung wird unter Rühren auf 70°C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 16 Stunden bei 70°C geführt. Im Anschluß wird das entstandene Produkt in einem vierfachen Überschuß Eiswasser gefällt. daraufhin 24 Stunden im Soxhlet mit Wasser extrahiert und bei 50°C im Vakuum getrocknet.
Eine anschließende Analyse der Zusammensetzung über 1H-NMR ergibt:

| | |
|---|---|
| Acrylsäure | 19 Mol-% |
| Natriumstyrolsulfonat | 5 Mol-% |
| Styrol | 76 Mol-% |

Aus diesen Werten ergibt sich ein Verhältnis der Carboxylatgruppen zu Sulfonatgruppen von 3.8.

### Beispiel 8:

In einer Stickstoffatmosphäre werden 310.6 g n-Butylmethacrylat, 10.4 g Acrylsäure und 14.8 g Natriumstyrolsulfonat in 500 ml Dimethylsulfoxid gelöst. Die Lösung wird unter Rühren auf 70 C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 16 Stunden bei 70 C geführt. Im Anschluß wird das entstandene Produkt in einem vierfachen Überschuß Eiswasser gefällt, daraufhin 24 Stunden im Soxhlet mit Wasser extrahiert und bei 50 C im Vakuum getrocknet.
Eine anschließende Analyse der Zusammensetzung über 1H-NMR ergibt:

| | |
|---|---|
| Acrylsäure | 8 Mol-% |
| Natriumstyrolsulfonat | 11 Mol-% |
| n-Butylmethacrylat | 81 Mol-% |

Aus diesen Werten ergibt sich ein Verhältnis der Carboxylatgruppen zu Sulfonatgruppen von 0.7.

### Herstellung von Membranen aus den erfindungsgemäßen Polymeren

### Beispiel 9:

Es wird eine 5%ige Dimethylsulfoxidlösung eines erfindungsgemäßen Polymeren hergestellt. Die Lösung wird in eine Petrischale gegossen, und der Probe wird das Lösemittel bei 80°C unter vermindertem Druck entzogen. Anschließend wird die Membran in Stücke von jeweils 0.5 cm² (für die Szintillationsmessung) bzw. von jeweils 2 cm² (für die ATP-Messungen) zerkleinert und 24 Stunden mit Wasser extrahiert. Vor den folgenden biologischen Untersuchungen werden die Membranstücke in einer Michaelis-Pufferlösung (pH = 7.33) drei mal jeweils drei Stunden gewaschen und bis zur weiteren Untersuchung bei -4°C aufbewahrt.

Die Adhäsion der Bakterienstämme Staphylococcus epidermidis, Streptococcus pyogenes und Staphylococcus aureus wurde an Polymerproben, erhalten nach den Beispielen 1 bis 8, untersucht. Die Messung der Bakterienadhäsion an diesen Proben durch das Szintillationsverfahren und die statische Bestimmung des ATP ergaben Bakterienadhäsionshemmungen von über 95 %, die dynamische Bestimmung des ATP ergab Bakterienadhäsionshemmungen von über 93 %. Herstellung von Polymerbeschichtungen durch Tauchen

### Beispiel 10:

Es wird eine 5%ige Methylethylketonlösung eines erfindungsgemäßen 5 Polymeren hergestellt. In diese Lösung wird eine 10 cm x 8 cm x 0.04 cm große Polyamidfolie für 10 Sekunden getaucht. Die Folie wird entnommen und 10 Stunden bei 50°C unter vermindertem Druck getrocknet. Anschließend wird die beschichtete Folie in Stücke von jeweils 0.5 cm² (für die Szintillationsmessung) bzw. von jeweils 2 cm² (für die ATP-Messungen) zerkleinert und 24 Stunden mit Wasser extrahiert. Vor den folgenden biologischen Untersuchungen werden die Membranstücke in einer Michaelis-Pufferlösung (pH = 7.33) drei mal jeweils drei Stunden gewaschen und bis zur weiteren Untersuchung bei -4°C aufbewahrt.

Die Adhäsion der Bakterienstämme Staphylococcus epidermidis, Streptococcus pyogenes und Staphylococcus aureus wurde an Polymerproben, erhalten nach den Beispielen 1 bis 8, untersucht. Die Messung der Bakterienadhäsion an diesen Proben durch das Szintillationsverfahren und die statische Bestimmung des ATP ergaben Bakterienadhäsionshemmungen von über 99 %, die dynamische Bestimmung des ATP ergab Bakterienadhäsionshemmungen von über 96 %.

### Beispiel 11:

Es wird eine 5%ige Acetonlösung eines erfindungsgemäßen Polymeren hergestellt. In diese Lösung wird eine 10 cm x 8 cm x 0.03 cm große Polyethylenfolie, deren Oberfläche zuvor durch 3-minütige Bestrahlung mit der 172 nm Strahlung eines Excimerstrahlers aktiviert wurde, für 15 Sekunden getaucht. Die Folie wird entnommen und 10 Stunden bei 50°C unter vermindertem Druck getrocknet. Anschließend wird die beschichtete Folie in Stücke von jeweils 0.5 cm² (für die Szintillationsmessung) bzw. von jeweils 2 cm² (für die ATP-Messungen) zerkleinert und 24 Stunden mit Wasser extrahiert. Vor den folgenden biologischen Untersuchungen werden die Membranstücke in einer Michaelis-Pufferlösung (pH = 7.33) drei mal jeweils drei Stunden gewaschen und bis zur weiteren Untersuchung bei -4°C aufbewahrt.

Die Adhäsion der Bakterienstämme Staphylococcus epidermidis, Streptococcus pyogenes und Staphylococcus aureus wurde an Polymerproben, erhalten nach den Beispielen 1 bis 8, untersucht. Die Messung der Bakterienadhäsion an diesen Proben durch das Szintillationsverfahren 5 und die statische Bestimmung des ATP ergaben Bakterienadhäsionshemmungen von über 98 %. die dynamische Bestimmung des ATP ergab Bakterienadhäsionshemmungen von über 96 %.

### Beispiel 12:

Es wird eine 5%ige Acetonlösung eines erfindungsgemäßen Polymeren hergestellt. In diese Lösung wird eine 10 cm x 8 cm x 0.04 cm große Polyetherblockamidfolie für 10 Sekunden getaucht. Die Folie wird entnommen und 10 Stunden bei 50°C unter vermindertem Druck getrocknet, Anschließend wird die beschichtete Folie in Stücke von jeweils 0.5 cm² (für die Szintillationsmessung) bzw. von jeweils 2 cm² (für die ATP-Messungen) zerkleinert und 24 Stunden mit Wasser extrahiert. Vor den folgenden biologischen Untersuchungen werden die Membranstücke in einer Michaelis-Pufferlösung (pH = 7.33) drei mal jeweils drei Stunden gewaschen und bis zur weiteren Untersuchung bei -4°C aufbewahrt.

Die Adhäsion der Bakterienstämme Staphylococcus epidermidis, Streptococcus pyogenes und Staphylococcus aureus wurde an Polymerproben. erhalten nach den Beispielen 1 bis 8, untersucht. Die Messung der Bakterienadhäsion an diesen Proben durch das Szintillationsverfahren und die statische Bestimmung des ATP ergaben Bakterienadhäsionshemmungen von über 98 %. die dynamische Bestimmung des ATP ergab Bakterienadhäsionshemmungen von über 95 %.

### Konditionierung der Proben aus erfindungsgemäßen Polymeren

### Beispiel 13:

Eine Membran gemäß Beispiel 9 und die mit den erfindungsgemäßen Polymeren beschichteten Folien gemäß den Beispielen 10 bis 12 werden durch 15-minütige Bestrahlung mit ultraviolettem Licht sterilisiert. Anschließend werden die so vorbehandelten Proben drei mal jeweils drei Stunden in einer 0.15 molaren Natriumchloridlösung gehalten. darauf drei Stunden mit destilliertem Wasser gewaschen. Im folgenden Reinigungsschritt werden sie drei mal jeweils drei Stunden in eine Phosphatpufferlösung folgender Zusammensetzung gelegt:

| | |
|---|---|
| CaCl₂ · H₂O | 0.132 g/l |
| KCl | 0.2 g/l |
| KH₂PO4 | 0.2 g/l |
| MgCl₂ · 6H₂O | 0.1 g/l |
| NaCl | 8 g/l |
| Na₂HPO₄ | 1.15 g/l |

Im Anschluß daran werden die Proben erneut 15 Minuten mit ultraviolettem Licht bestrahlt. Die so vorhandenen Proben werden ca. 16 Stunden bei 37°C in einer DMEM-Lösung (Dulbecco's Modified Eagles Medium) gelagert. Abschließend werden die Proben weitere 16 Stunden in einer mit Antibiotika. L-Glutamin und 10 Vol.-% eines fötalen Kälberserums versetzten DMEM-Lösung bei 37°C und einer Atmosphäre von 5 % CO² und 95 % Luft gehalten.

Die nach den Beispielen 1, 2 und 5 hergestellten erfindungsgemäßen Polymeren wurden entweder zu einer Membran (Beispiel 9) verarbeitet oder auf Standardpolymere (Beispiele 10 bis 12) aufgebracht. Anschließend wurde eine Konditionierung dieser Proben nach Beispiel 13 durchgeführt und die Zellproliferation gemäß dem beschriebenen Verfahren bestimmt.

Die Inhibierung der Zellproliferation überstieg bei allen Proben 98%.

## Patentansprüche

1. Wasserunlösliche, bakteriophobe und gegebenenfalls zellproliferationsinhibierende Polymeren, erhältlich durch readikalische Copolymerisation von
(c) einem oder mehreren Carboxylgruppen- und/oder Carboxylatgruppen-haltigen, aliphatisch ungesättigten Monomeren oder den entsprechend funktionalisierten Derivaten dieser Monomeren als Komponente I mit
(d) einem oder mehreren Sulfonsäuregruppen- und/oder Sulfonatgruppen-haltigen, aliphatisch ungesättigten Monomeren oder den entsprechend funktionalisierten Derivaten dieser Monomeren als Komponente II und
(b) einer Komponente III, die ein weiteres aliphatisch ungesättigtes Monomer oder mehrere weitere aliphatisch ungesättigte Monomere enthält,
wobei die entsprechend funktionalisierten Derivate nach der Copolymerisation zumindest an der Oberfläche in Carboxyl- und/oder Carboxylatgruppen bzw. Sulfonsäure- und/oder Sulfonatgruppen überführt werden und das Verhältnis der im Polymer enthaltenen Carboxyl- und/oder Carboxylatgruppen zu Sulfonsäure- und/oder Sulfonatgruppen 0.4 bis 2 beträgt.

2. Polymere nach Anspruch 1, **dadurch gekennzeichnet, daß** ein bifunktionelles, Carboxylund/oder Carboxylatgruppen sowie Sulfonsäure- und/oder Sulfonatgruppen-haltiges, aliphatisch ungesättigtes Monomer als Komponente (I + II) verwendet wird.

3. Polymere nach den Ansprüchen 1 und 2, **dadurch gekennzeichnet, daß** die Summe der Anteile von Komponente I und Komponente II 0.5 bis 30 Mol-% des Polymeren beträgt.

4. Verfahren zur Herstellung der wasserunlöslichen, bakteriophoben und gegebenenfalls zellproliferationsinhibierenden Polymeren B gemäß den Ansprüchen 1 bis 3, **dadurch gekennzeichnet, daß** man
(c) ein oder mehrere Carboxylgruppen- und/oder Carboxylatgruppen-haltige, aliphatisch ungesättigte Monomere oder entsprechend funktionalisierte Derivate dieser Monomeren als Komponente I mit
(d) einem oder mehreren Sulfonsäuregruppen- und/oder Sulfonatgruppen-haltigen, aliphatisch ungesättigten Monomeren oder entsprechend funktionalisierten Derivaten dieser Monomeren als Komponente II und
(b) einer Komponente III, die ein aliphatisch ungesättigtes Monomer oder mehrere aliphatisch ungesättigte Monomere enthält,
radikalisch copolymerisiert, wobei die entsprechend funktionalisierten Derivate nach der Copolymerisation zumindest an der Oberfläche in Carboxyl- oder Carboxylat- bzw. Sulfonsäure- oder Sulfonatgruppen überführt werden und das Verhältnis der im Polymer enthaltenen Carboxyl- und/oder Carboxylatgruppen zu Sulfonsäure- und/oder Sulfonatgruppen 0.4 bis 2 beträgt.

5. Verfahren nach Anspruch 4, **dadurch gekennzeichnet, daß** ein bifunktionelles, Carboxylund/oder Carboxylat- sowie Sulfonsäure und/oder Sulfonatgruppen-haltiges, aliphatisch ungesättigtes Monomer als Komponente (I + II) verwendet wird.

6. Verfahren nach den Ansprüchen 4 und 5, dadurch gekennzeichnt, daß die Summe der Anteile von Komponente I und Komponente II 0.5 bis 30 Mol-% des Polymeren beträgt.

7. Verfahren zur Herstellung von Erzeugnissen mit bakteriophober und gegebenenfalls zellproliferationsinhibierender Oberflächen, **gekennzeichnet durch** die Verwendung von Polymeren nach den Ansprüchen 1 bis 3.

8. Verfahren nach Anspruch 7, **dadurch gekennzeichnet, daß** die Erzeugnisse Textilien, Möbel und Geräte, Fußböden, Wand- und Deckenflächen, Rohre oder Schläuche, Verpackungen, Fensterrahmen oder medizintechnische Artikel sind.

9. Verfahren zur Herstellung von Erzeugnissen mit einer bakteriophoben und gegebenenfalls zellproliferationsinhibierenden Beschichtung, **dadurch gekennzeichnet, daß** man ein Polymer nach den Ansprüchen 1 bis 3 als Beschichtungsmittel verwendet.

10. Verfahren nach Anspruch 9, **dadurch gekennzeichnet, daß** die Erzeugnisse Textilien, Möbel und Geräte, Fußböden, Wand- und Deckenflächen, Rohre und Schläuche, Lagerbehälter, Verpackung, Fensterrahmen oder medizintechnische Artikel aus Kunststoffen, Keramiken oder Metallen sind.

11. Verfahren nach Anspruch 10, **dadurch gekennzeichnet, daß** die medizintechnischen Artikel Katheter, Drainagen oder Intraokularlinsen sind.

12. Erzeugnisse mit einer bakteriophoben und gegebenenfalls zellproliferationsinhibierenden Oberfläche aus wasserunlöslichen, bakteriophoben, Carboxyl- und/oder Carboxylatgruppen sowie Sulfonsäure- und/oder Sulfonatgruppen enthaltenden Polymeren gemäß den Ansprüchen 1 bis 3.

13. Erzeugnisse nach Anspruch 12, **dadurch gekennzeichnet, daß** die Erzeugnisse Textilien, Möbel und Geräte, Fußböden, Wand- und Deckenflächen, Rohre, Lagerbehälter, Verpackungen, Fensterrahmen und medizintechnische Artikel sind.

14. Erzeugnisse mit einer bakteriophoben Beschichtung aus wasserunlöslichen, bakteriophoben Carboxyl- und/oder Carboxylatgruppen sowie Sulfonsäure- und/oder Sulfonatgruppen enthaltenden Polymeren gernäß den Ansprüchen 1 bis 3.

15. Erzeugnisse nach Anspruch 14, **dadurch gekennzeichnet, daß** die Erzeugnisse Textilien, Möbel und Geräte, Fußböden, Wand- und Deckenflächen, Rohre, Lagerbehälter, Verpackungen, Fensterrahmen und medizintechnische Artikel aus Kunststoffen, Keramiken oder Metallen sind.

16. Erzeugnisse nach Anspruch 15, **dadurch gekennzeichnet, daß** die medizintechnischen Artikel Katheter, Drainagen oder Intraokularlinsen sind.

## Claims

1. A water-insoluble, bacteriophobic and optionally cell proliferation-inhibiting polymer obtainable by free-radical copolymerization of
(c) one or more aliphatically unsaturated monomers which contain carboxyl groups and/or carboxylate groups, or the correspondingly functionalized derivatives of these monomers, as component I, with
(d) one or more aliphatically unsaturated monomers containing sulphonic acid groups and/or sulphonate groups, or the correspondingly functionalized derivatives of these monomers, as component II, and
(b) a component III which comprises a further aliphatically unsaturated monomer or a plurality of further aliphatically unsaturated monomers,
the correspondingly functionalized derivatives being converted if desired, after copolymerization and at least on the surface, into carboxyl or carboxylate groups or sulphonic acid or sulphonate groups, respectively, and the ratio of the carboxyl and/or carboxylate groups present in the polymer to sulphonic acid and/or sulphonate groups being from 0.4 to 2.

2. A polymer according to claim 1, **characterized in that** a bifunctional, aliphatically unsaturated monomer containing carboxyl and/or carboxylate groups and sulphonic acid and/or sulphonate groups is used as component (I + II).

3. A polymer according to either of claims 1 and 2, 5 **characterized in that** the sum of the proportions of component I and component II is from 0.5 to 30 mol-% of the polymer.

4. A process for preparing a water-insoluble, bacteriophobic and optionally cell proliferation-inhibiting polymer B according to any of claims 1 to 3, **characterized in that** it comprises subjecting
(c) one or more aliphatically unsaturated monomers which contain carboxyl groups and/or carboxylate groups, or correspondingly functionalized derivatives of these monomers, as component I, to free-radical copolymerization with
(d) one or more aliphatically unsaturated monomers containing sulphonic acid groups and/or sulphonate groups, or correspondingly functionalized derivatives of these monomers, as component II, and
(b) a component III which comprises an aliphatically unsaturated monomer or a plurality of aliphatically unsaturated monomers,
the correspondingly functionalized derivatives being converted if desired, after copolymerization and at least on the surface, into carboxyl or carboxylate groups or sulphonic acid or sulphonate groups, respectively, and the ratio of the carboxyl and/or carboxylate groups present in the polymer to sulphonic acid and/or sulphonate groups being from 0.4 to 2.

5. A process according to claim 4, **characterized in that** a bifunctional, aliphatically unsaturated monomer containing carboxyl and/or carboxylate groups and sulphonic acid and/or sulphonate groups is used as component (I + II).

6. A process according to either of claims 4 and 5, **characterized in that** the sum of the proportions of component I and component II is from 0.5 to 30 mol-% of the polymer.

7. A process for producing an article having a bacteriophobic and optionally cell proliferation-inhibiting surface, **characterized by** the use of a polymer according to any of claims 1 to 3.

8. A process according to claim 7, **characterized in that** the article is selected from the group consisting of textiles, furniture and instruments, flooring, wall and ceiling surfaces, tubes and hoses, packaging, window frames and medical articles.

9. A process for producing an article having a bacteriophobic and optionally cell proliferation-inhibiting coating, **characterized in that** it comprises using a polymer according to any of claims 1 to 3 as coating material.

10. A process according to claim 9, **characterized in that** the article is selected from the group consisting of textiles, furniture and instruments, flooring, wall and ceiling surfaces, tubes and hoses, storage containers, packaging, window frames and medical articles made from plastics, ceramics or metals.

11. A process according to claim 10, **characterized in that** the medical articles are catheters, drainage tubes or intraocular lenses.

12. An article having a bacteriophobic and optionally cell proliferation-inhibiting surface, comprising a water-insoluble, bacteriophobic polymer which contains carboxyl and/or carboxylate groups and sulphonic acid and/or sulphonate groups, according to any of claims 1 to 3.

13. An article according to claim 12, **characterized in that** the article is selected from the group consisting of textiles, furniture and instruments, flooring, wall and ceiling surfaces, tubes, storage containers, packaging, window frames and medical articles.

14. An article having a bacteriophobic coating comprising a water-insoluble, bacteriophobic polymer which contains carboxyl and/or carboxylate groups and sulphonic acid and/or sulphonate groups, according to any of claims 1 to 3.

15. An article according to claim 14, **characterized in that** the article is selected from the group consisting of textiles, furniture and instruments, flooring, wall and ceiling surfaces, tubes, storage containers, packaging, window frames and medical articles made from plastics, ceramics or metals.

16. An article according to claim 15, **characterized in that** the medical articles are catheters, drainage tubes or intraocular lenses.

## Revendications

1. Polymères insolubles dans l'eau, bactériophobes et éventuellement inhibiteurs de la prolifération cellulaire, que l'on peut obtenir par copolymérisation par voie radicalaire de
c) un ou plusieurs monomères non saturés aliphatiquement, contenant des groupes carboxyle et/ou carboxylate ou les dérivés fonctionnalisés d'une manière correspondante de ces monomères, en tant que composant I avec,
d) des monomères non saturés aliphatiquement, contenant un ou plusieurs groupes acide sulfonique et/ou groupes sulfonate, ou les dérivés fonctionnalisés d'une manière correspondante de ces monomères en tant que composant II, et
b) avec un composant III qui renferme dans un autre monomère non saturé aliphatiquement ou dans plusieurs monomères supplémentaires non saturés aliphatiquement,
les dérivés fonctionnalisés d'une manière correspondante étant convertis après la copolymérisation au moins à la surface en groupes carboxyle et/ou groupes carboxylate ou acide sulfonique et/ou groupes sulfonate, et le rapport des groupes carboxyle et/ou des groupes carboxylate contenus dans le polymère aux groupes acide sulfonique et/ou sulfonate étant de 0,4 à 2.

2. Polymères selon la revendication 1,
**caractérisés en ce que**
comme composant I on utilise un monomère contenant des groupes carboxyle et/ou carboxylate ainsi que des groupes acide sulfonique et/ou sulfonate en tant que composant (I + II).

3. Polymères selon la revendication 1 et la revendication 2,
**caractérisés en ce que**
la somme des fractions de composant I et de composant II s'élève de 0,5 à 30 % molaire du polymère.

4. Procédé de préparation des polymères B insolubles dans l'eau, bactériophobes et éventuellement inhibiteurs de la prolifération cellulaire conformément aux revendications 1 à 3,
**caractérisé en ce qu'**
on copolymérise par voie radicalaire,
c) des monomères non saturés aliphatiquement, contenant un ou plusieurs groupes carboxyle et/ou carboxylate ou des dérivés fonctionnalisés d'une manière correspondante de ces monomères en tant que composant I avec,
d) un ou plusieurs monomères non saturés aliphatiquement contenant des groupes acide sulfonique et/ou sulfonate ou des dérivés fonctionnalisés d'une manière correspondante de ces monomères, en tant que composant II, et
b) avec un composant III qui renferme un monomère non saturé aliphatiquement ou plusieurs monomères non saturés aliphatiquement, les dérivés fonctionnalisés d'une manière correspondante étant convertis après la copolymérisation au moins à la surface, en groupes carboxyle ou carboxylate ou acide sulfonique ou sulfonate, et le rapport des groupes carboxyle et/ou carboxylate contenus dans le polymère aux groupes acide sulfonique et/ou sulfonate étant de 0,4 à 2.

5. Procédé selon la revendication 4,
**caractérisé en ce qu'**
on utilise un monomère non saturé aliphatiquement, bifonctionnel, contenant des groupes carboxyle et/ou carboxylate, ainsi qu'acide sulfonique et/ou sulfonate, comme composant (I + II).

6. Procédé selon la revendication 4 et la revendication 5,
**caractérisé en ce que**
la somme des fractions du composant I et du composant II s'élève de 0,5 à 30 % molaire de polymère.

7. Procédé de préparation de produits ayant des surfaces bactériphobes et éventuellement inhibitrices de la prolifération cellulaire, **caractérisé par** l'utilisation de polymères selon les revendications 1 à 3.

8. Procédé selon la revendication 7,
**caractérisé en ce que**
les produits sont des articles textiles, des meubles et des appareils, des planchers, des surfaces de paroi et de plafond, des tubes et des tuyaux, des emballages, des cadres de fenêtre ou des articles de technique médicale.

9. Procédé de préparation de produits ayant un revêtement bactériophobe et éventuellement inhibiteur de la prolifération cellulaire,
**caractérisé en ce qu'**
on utilise comme agent de revêtement un polymère selon l'une des revendications 1 à 3.

10. Procédé selon la revendication 9,
**caractérisé en ce que**
les produits sont des articles textiles, des meubles et des appareils, des planchers, des surfaces de paroi et de plafond, des tubes et des tuyaux, des récipients de stockage, des emballages, des cadres de fenêtre ou des articles de technique médicale en matières plastiques, céramiques ou en métaux.

11. Procédé selon la revendication 10,
**caractérisé en ce que**
les articles de technique médicale sont des cathéters, des drainages ou des lentilles intraoculaires.

12. Produits ayant des surfaces bactériophobes et éventuellement inhibitrices de la prolifération cellulaire, en polymères contenant des groupes carboxyle et/ou carboxylate ainsi que des groupes acide sulfonique et/ou sulfonate conformément aux revendications 1 à 3.

13. Produits selon la revendication 12,
**caractérisé en ce que**
les produits sont des articles textiles, des meubles et des appareils, des planchers, des surfaces de paroi et de plafond, des tubes et des tuyaux, des récipients de stockage, des emballages, des cadres de fenêtre et des articles de technique médicale.

14. Produits ayant un revêtement bactériophobe en polymères insolubles dans l'eau, bactériophobes contenant des groupes carboxyle et/ou carboxylate ainsi que des groupes acide sulfonique et/ou sulfonate conformément aux revendications 1 à 3.

15. Produits selon la revendication 14,
**caractérisés en ce que**
les produits sont des articles textiles, des meubles et des appareils, des planchers, des surfaces de mur et de plafond, des tubes, des récipients de stockage, des emballages, des cadres de fenêtre et des articles de technique médicale, en matières plastiques, céramiques ou en métaux.

16. Produits selon la revendication 15,
**caractérisé en ce que**
les articles de technique médicale sont des cathéters, des drainages ou des lentilles intraoculaires.
